# EUROPEAN PATENT SPECIFICATION

(11) **EP 2 092 948 B1**
(45) Date of publication and mention of the grant of the patent: **07.05.2014**
(21) Application number: 07831809.4
(22) Date of filing: 14.11.2007
(51) Int. Cl.: A61M 5/31

(54) **INJECTION NOZZLE CAP**
INJEKTIONSDÜSENKAPPE
CAPUCHON DE BUSE D'INJECTION

(30) Priority: 16.11.2006 JP 2006310750
(43) Date of publication of application: 26.08.2009
(73) Proprietor: Daikyo Seiko, LTD., Tokyo 131-0031 (JP)
(72) Inventor: SUDO, Masamichi, Sumida-ku Tokyo 131-0031 (JP); KAWACHI, Yasushi, Sumida-ku Tokyo 131-0031 (JP)
(74) Representative: Hartz, Nikolai
(86) International application number: PCT/JP2007/072079
(87) International publication number: WO 2008/059863

(56) References cited:
- WO-A1-2005/032627
- DE-C- 817 097
- FR-A- 1 061 943
- JP-A- 08 275 984
- JP-A- 08 322 906
- JP-A- 2002 153 539
- JP-A- 2002 165 861

## Description

### Technical Field

This invention relates to an injection nozzle cap (which may hereinafter be simply called "cap") for a pre-filled syringe (which may hereinafter be simply called "syringe") having a female luer-lock fitting, and more specifically to a syringe cap which permits easy capping and hardly falls off, for example, upon filling a drug solution.

### Background Art

In recent years, there are usedpre-filled syringes (syringes usable as containers) with drug solutions or nutrient infusions filled and sealed beforehand in syringe barrels of syringes. The use of these syringes makes it possible to avoid false handling, contamination by mixed foreign matter, damages to syringe needles and the like upon drawing drug solutions from containers such as ampoules or vials into syringes, and also to save the labor and time needed to draw thixotropic (thixotropy-possessing) drug solutions or the like in case of emergencies.

A pre-filled syringe is provided on a head portion thereof with a female luer-lock fitting formed to facilitate the connection of an extension tube, three-way cock or syringe needle for mixing a drug in an infusion solution or the like or injecting a high-viscosity drug or the like.

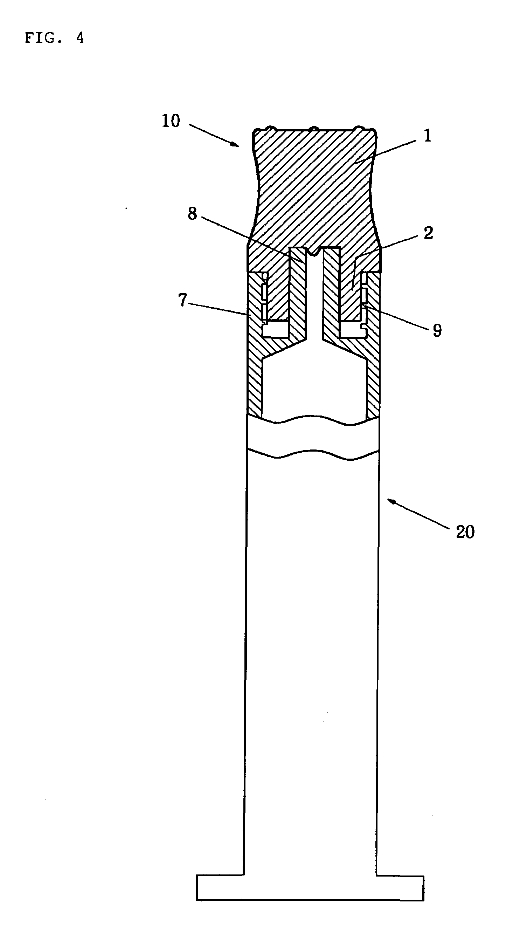

With reference to FIG. 4, the female luer-lock fitting of the pre-filled syringe 20 will be described. The female luer-lock fitting 7 surrounds an injection nozzle 8, and is comprised of a cylinder which is lower in height than the injection nozzle 8 and is concentric with the injection nozzle 8. A helical ridge 9 is formed on an inner peripheral surface of the cylinder. An extension tube (not shown), which is to be connected to the fitting, is provided at an free end thereof with a cylindrical connection member to be brought into threaded engagement with the nozzle 8 and female luer-lock fitting 7 of the syringe 20. By threadingly fitting this connection member into a space between the injection nozzle 8 and the female luer-lock fitting 7, the syringe 20 and the extension tube can be connected together.

Upon producing a pre-filled syringe, a cap 10 is firstly fitted on the injection nozzle 8 and female luer-lock fitting 7. This fitting operation of the cap 10 is generally called "capping". The syringe 20 with the cap 10 capped thereon is sterilized with heat, steam, electron beam or the like, and at a pharmaceutical company or the like, a drug solution is filled and sealed in the syringe 20.

Employed as the cap 10 for the conventionally-employed syringe 20 provided with the female luer-lock fitting 7 is a rubber plug or closure having a cylindrical leg portion 2, which is to be inserted between the female luer-lock fitting 7 and the nozzle 8, and a base portion 1 holding the cylindrical leg portion 2 thereon.

Upon producing pre-filled syringes, such caps 10 are continuously and automatically capped at high speed on the syringes. The capping is, therefore, required to be easy. On the other hand, the syringes 20 with the caps 10 applied thereon are conveyed on and along a production line at a pharmaceutical company or the like with the caps 10 directed downwards, and a drug solution is continuously filled in the syringes 20. It is, therefore, not permissible for these caps 10 to fall off, for example, during the filling of the drug solution. As described above, the mutually-contradictory performances of easy capping and fall-off prevention are required for the caps 10.

For such mutually-contradictory requirements as described above, it is proposed in Patent Document 1 to arrange a helical ridge on the outer wall of the cylindrical leg portion 2 of the cap and to bring the helical ridge into engagement with a helical ridge 9 formed on the inner wall of the female luer-lock fitting 7. The above-described proposed cap can be surely capped by manually bringing it into threaded engagement with the female luer-lock fitting, and upon filling a drug solution, the possibility of fall-off of the cap is low, see e.g. WO 2005/032627.
Patent Document 1: JP-A-2002-153539

### Disclosure of the Invention

### Problem to Be Solved by the Invention

When performing automated, high-speed capping of the caps, however, it is difficult to bring the helical ridge on each cap into threaded engagement with the helical ridge 9 on the corresponding female luer-lock fitting. The group of syringes so capped, therefore, includes those capped incompletely in a state that the helical ridges are not in mutual threaded engagement, thereby developing a problem that upon filling a drug solution into the syringes or on a conveyance line before or after the filing, the caps may fall off from the syringes and the drug solution may hence leak out.

An object of the present invention is, therefore, to provide a nozzle cap for a pre-filled syringe, which can be surely and automatically capped at high speed on a syringe having a female luer-lock fitting without occurrence of its fall-off, for example, upon filling a drug solution.

### Means for Solving the Problem

The above-described object can be achieved by the present invention to be described hereinafter. Described specifically, the present invention provides an injection nozzle cap (10) for being fittedly inserted in a space between an injection nozzle of a syringe having a cylindrical, female luer-lock fitting and the female luer-lock fitting, characterized in that the injection nozzle cap (10) comprises a base portion (1) and a cylindrical leg portion (2) extending downwardly of the base portion (1), and the cylindrical leg portion (2) is provided on an outer wall thereof with a substantially annular, bulged portion and/or a ring.

In the above-described cap (10) according to the present invention, an inner diameter (β) of a distal end portion of the cylindrical leg portion (2) may preferably be formed greater than an inner diameter (α) of a boundary portion between the cylindrical leg portion (2) and the base portion (1); and the cylindrical leg portion (2) may preferably be coated on at least an inner surface and/or outer surface thereof with a fluorinated resin film.

### Advantageous Effects of the Invention

According to the present invention, there can be provided a nozzle cap for a pre-filled syringe, which can be surely and automatically capped at high speed on a syringe having a female luer-lock fitting without occurrence of its fall-off, for example, upon filling a drug solution.

### Best Modes for Carrying out the Invention

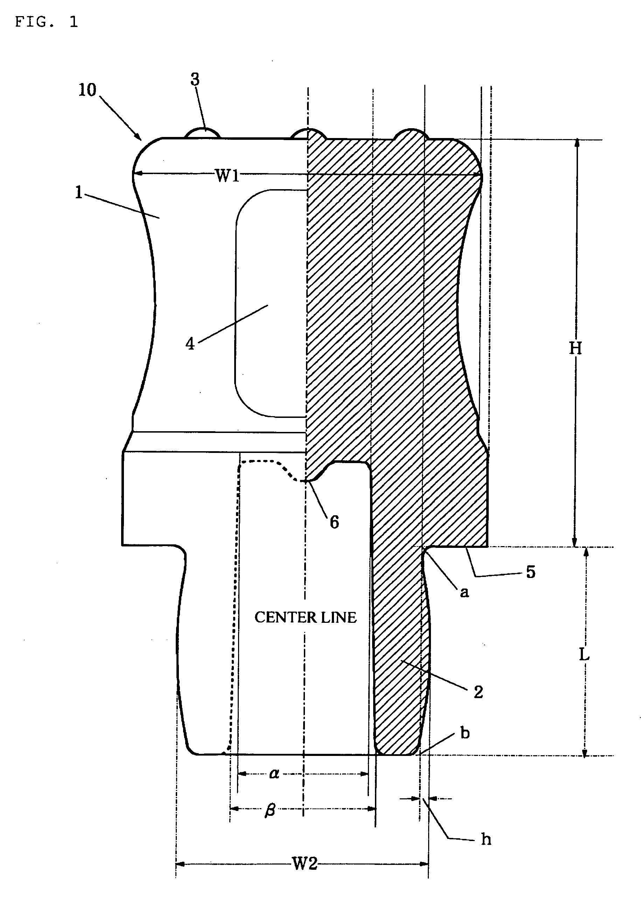

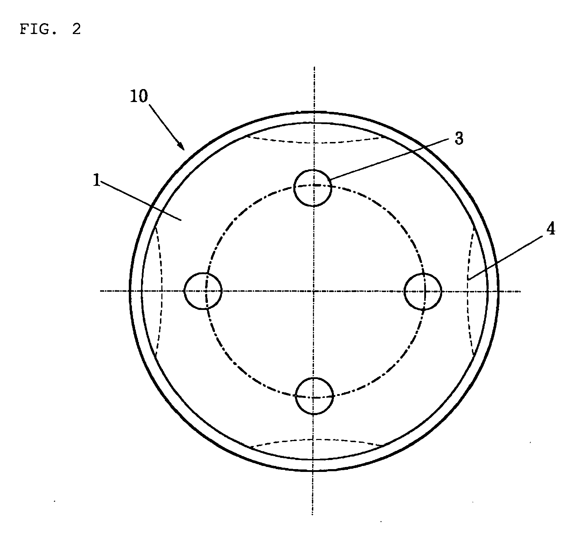

The present invention will next be described in further detail based on best modes for carrying out the invention. FIG. 1 is a view illustrating a cap according to the present invention, in which the left half is a side view and the right half is a cross-sectional view. FIG. 2 is a plan view of the cap of FIG. 1. As depicted in FIG. 1 and FIG. 2, the cap 10 according to the present invention is comprised of a rubber-made, solid, circular or polygonal cylindrical, rubber-made base portion 1 and a rubber-made, cylindrical leg portion (which may hereinafter be simply called "leg portion") 2 formed integrally with and extending downwardly from the base portion 1. The base portion 1 is formed solid to provide the entire cap 10 with a certain degree of rigidness.

The base portion 1 depicted in FIG. 1 and FIG. 2 is provided on a top portion thereof with some small convexities 3. These convexities 3 are intended to prevent plural caps 10 from sticking each other at the time of washing, drying or the like in the course of their production. However, these convexities are not essential in the present invention, and constitute a preferred embodiment. On an outer periphery of the base portion 1, four recessed parts 4 are also formed. As illustrated in FIG. 3, these recessed parts 4 are arranged to permit easy detachment of the cap 10 from the syringe 20 by a thumb upon using the syringe 20. These recessed parts 4 are not essential in the present invention, and constitute another preferred embodiment.

The leg portion 2, which constitutes the cap 10 according to the present invention, is formed in the shape of a cylinder in continuation with a lower wall of the base portion 1 such that the cylinder is open at a distal end thereof. The lower wall 5 of the base portion 1, said lower wall 5 extending to an outer upper part of the outer periphery of the leg portion 2, is located at a position where the base portion 1 is in contact with a top wall of a female luer-lock fitting 7 as shown in FIG. 4, and an outer wall of the leg portion 2, said outer wall downwardly extending from the lower wall 5, is formed in a shape that the outer wall gently bulges out in the form of a circular arc the peak of which is located at substantially the middle between the top and bottom of the outer wall.

The degree of the above-described bulge may be in the range of h/L = 0.5/6.0 to 1.0/6.0, preferably h/L = 0.6/6.0 to 0.9/6.0 in which L represents the length of a line a-b (a: a start point at the position of connection between the base portion 1 and the leg portion 2; b: an end point at the distal end of the leg portion 2) and h represents the height from the line a-b to the ridge of the bulge. An h/L value greater than the above-described range is not preferred for the automated capping of the cap 10 on the syringe 20, while an h/L value smaller than the above-described range may lead to fall-off of the cap 10 from the syringe 20, for example, upon filling a drug solution.

It is to be noted that instead of the above-described bulge shape, the bulged portion may also be in the shape of a ring formed on only a part of the leg portion in the present invention. It is the most important characteristic feature of the present invention that the leg portion is not provided with any helical ridge threadedly engageable with the helical ridge 9 formed on the female luer-lock fitting 7 of the syringe 20 but is provided with the bulged portion or ring which does not threadedly engage the helical ridge 9. Owing to such a construction, it is possible to achieve the object of the present invention that is to automatically cap the cap 10 at high speed on the syringe 20 and to prevent the cap 10 from falling off from the syringe.

Further, two or more bulged portions or rings can be arranged in the present invention. However, the greater the number of bulged portions or rings, the higher the capping resistance of the cap 10, resulting in the observation of a phenomenon called "a cap lift" that a clearance is formed between the lower wall 5 of the base portion 1 of the cap 10 and the top wall of the female luer-lock fitting 7 after the capping. Accordingly, the number of bulged portion (s) or ring (s) can preferably be 1 or 2.

The position of the ridge of the bulged portion or ring may preferably be around the middle of the outer wall of the leg portion 2 in many instances. This position can be selectively set as desired depending on the shape of the cap 10 and the dimensions and shapes of the female luer-lock fitting 7 and nozzle 8 of the syringe 20. For example, the setting of the position of the ridge of the bulged portion or ring on a side close to the base portion 1 is highly effective for the inhibition of a lift of the cap, while the setting of the ridge on the side of the distal end of the leg portion 2 is highly effective for the reduction of the capping resistance of the cap.

It is to be noted that the ridge of the bulged portion or ring may preferably be formed over the entire circumference of the leg portion 2. For air bleeding or the like upon capping, however, the ridge may be formed intermittently. In such a case, the parts at which no ridge is formed (interrupted parts) may account preferably for less than 40%, more preferably for less than 20% of the entire circumference of the bulged portion or ring. Preferably, the bulged portion or ring may be formed such that it extends out in a direction perpendicular to the capping direction of the cap 10. Such construction is effective in stabilizing the state of engagement between the helical ridge 9 of the female luer-lock fitting 7 and the bulged portion or ring of the cap 10 when the cap 10 is capped on the syringe 20, and hence in reducing a variation in the capping resistance or fall-off resistance of the cap 10.

In the present invention, an open, distal end portion of the cylindrical leg portion 2 may preferably be formed with an inner diameter (β) greater than the inner diameter (α) of a boundary portion between the cylindrical leg portion 2 and the base portion 1. In other words, the leg portion 2 may preferably be in a shape that it is somewhat wider at its opening than at the boundary portion. As the leg portion 2 somewhat flares out at the opening, the fitting insertion of the cap 10 into the syringe 20 causes the opening to shrink so that upon filling a drug solution, the fall-off of the cap 10 from the syringe 20 can be more preferably prevented owing to the resilience of the leg portion 2 that it tries to recover its original shape.

The degree of the flare of the opening differs depending on the length of the leg portion 2, the friction resistance between the material of the cap 10 and that of the syringe 20, and the like. Preferably, however, the opening may have a flare a little smaller than 6/100 which is a taper of the nozzle 8. Despite the somewhat flaring configuration of the opening of the leg portion 2 as described above, no problem arises upon capping because the outer wall of the distal end of the leg 2 is formed with roundness (a curved surface).

Further, the leg portion 2 is fittedly inserted in the space between the nozzle 8 of the syringe 20 and the female luer-lock fitting 7, an internal space of the cylindrical leg portion 2 is configured to surround the nozzle 8, and a downward protuberance 6 is arranged on a ceiling portion inside the leg portion 2 (namely, at the boundary with the base portion 1). This protuberance 6 is formed such that it just closes up a bore of the nozzle 8 when the cap 10 is fittedly inserted in the space between the nozzle 8 of the syringe 20 and the female luer-lock fitting 7.

The inner diameter of the leg portion 2 of the cap 10 according to the present invention is slightly smaller than the outer diameter of the nozzle 8 of the syringe 20. When the nozzle 8 of the syringe 20 is inserted into the leg portion 2, the leg portion 2 is caused to flare out so that sealing performance for the nozzle 8 is maintained. Further, the outer wall of the leg portion 2 comes into contact under pressure with a cylindrical inner wall of the female luer-lock fitting 7. This pressure contact also takes part in the maintenance of the sealing performance of the cap 10, and moreover, is effective in preventing the cap 10 from falling off.

The cap 10 according to the present invention can most prominently bring about the advantageous effects of the present invention when the leg portion 2 is coated on at least its outer surface, preferably on its entire surfaces with a fluorinated resin film of approx. 10 to 200 µm thickness (not shown) concurrently with the molding of the cap 10. In general, the inner surface and outer surface of the leg portion 2 may come into contact with a drug solution filled in the syringe 20, so that the fluorinated resin film is laminated to eliminate the possibility of mixing of toxic substances into the drug solution from the rubber-made cap 10 by its contact with the drug solution.

When the fluorinated resin film is laminated on the surface of the leg portion 2, its lubricity facilitates the automated capping of the cap 10 but renders the cap 10 prone to fall off from the syringe 20 upon filling the drug solution. Owing to the above-described construction of the present invention, the present invention can make the cap 10 free of such a problem upon automated capping and also resistant to fall-off upon filling the drug solution even when the cap 10 is provided with the fluorinated resin film laminated on the leg portion 2.

The dimensions of the cap 10 according to the present invention varies depending on the dimensions of the syringe 20. For example, referring to FIG. 1, the height H of the base portion 1 can be 8 to 20 mm or so (15 mm in the illustrated embodiment), the width (diameter) W1 of the base portion 1 can be 8 to 13 mm or so (9.75 mm in the illustrated embodiment), the height and diameter of each convexity 3 arranged on the base portion 1 can be 0.2 to 0.3 mm or so and 0.7 to 1.3 mm or so, respectively (0.25 mm and 1.0 mm, respectively, in the illustrated embodiment), and the depth and size of each recessed part 4 arranged on the base portion 1 can be 0.4 to 0.8 mm or so and 3 to 5 × 5 to 7 mm or so, respectively (0.6 mm and 4 × 6.5 mm, respectively, in the illustrated embodiment).

On the other hand, the length L of the leg portion 2 can be 3 to 10 mm or so (6 mm in the illustrated embodiment), the outer diameter W2 of the leg portion 2 can be 10 to 13 mm (11.2 mm in the illustrated embodiment), the depth of the internal space (nozzle-receiving portion) of the leg portion 2 can be 7 to 10 mm or so (8.5 mm in the illustrated embodiment), the inner diameters α and β in the internal space of the leg portion 2 can be 3.0 to 5.0 mm or so and 3.5 to 6.0 mm, respectively (3.7 mm and 4.1 mm, respectively, in the illustrated embodiment), the h in the leg portion 2 can be 0.5 to 1.0 mm or the like (0.8 mm in the illustrated embodiment), and the thickness of the unillustrated fluorinated resin film coated on the entirety of the leg portion 2 can be 10 to 200 µm (50 µm in the illustrated embodiment). It is to be noted that the cap 10 is molded in its entirety of chlorinated butyl rubber.

Except for the possession of such construction as described above, the cap 10 according to the present invention can be produced in a similar manner as the caps for the conventional pre-filled syringes. The rubber material for use in the production of the cap according to the present invention may preferably be butyl rubber or chlorinated butyl rubber. Including additives, those employed in the production of conventional caps and also of conventional rubber plugs or closures for drugs and medical care are all usable, and no particular limitation is imposed thereon. To prevent the surfaces of the leg portion of the cap and the content of the syringe from coming into direct contact with each other, the surfaces can be coated with high molecular weight polyethylene films or films of a resin such as a fluorinated resin similar to the conventional caps and the conventional rubber plugs or closures for drugs and medical care.

Likewise, the surfaces of the base portion 1 can also be coated with such films. These films are not applied under the premise of the contact of the surfaces with a drug solution, but are coated to overcome the tackiness which the rubber possesses. The films for this purpose can be those of a general-purpose resin such as, for example, polyethylene or polypropylene.

### Industrial Applicability

According to the present invention as described above, it is possible to provide a cap for a pre-filled syringe, which can be surely and automatically capped on a syringe having a female luer-lock fitting and does not fall off upon filling a drug solution.

### Brief Description of the Drawings

[FIG. 1] A partial side and partial cross-sectional view illustrating a cap according to the present invention.
[FIG. 2] A plan view of the cap according to the present invention.

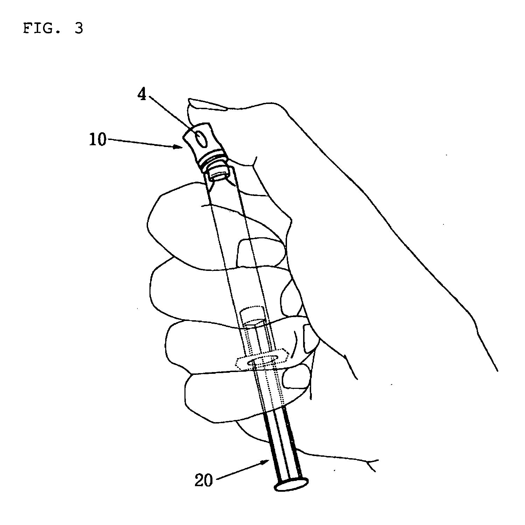

[FIG. 3] A view illustrating the manner of use of the cap according to the present invention.
[FIG. 4] A view illustrating the relation between a syringe and a cap.

### Legend

1: Base portion
2: Leg portion
3: Convexities
4: Recessed parts
5: Lower wall
6: Protuberance
7: Female luer-lock fitting
8: Injection nozzle
9: Helical ridge
10: Cap
20: Syringe

## Claims

1. An injection nozzle cap (10) for being fittedly inserted in a space between an injection nozzle of a syringe having a cylindrical, female luer-lock fitting and a female luer-lock fitting, said injection nozzle cap (10) comprises a base portion (1) and a cylindrical leg portion (2) extending downwardly of said base portion (1), **characterized in that** said cylindrical leg portion (2) is provided on an outer wall thereof with a substantially annular, bulged portion and/or a ring.

2. An injection nozzle cap (10) according to claim 1, wherein an inner diameter (β) of a distal end portion of said cylindrical leg portion (2) is formed greater than an inner diameter (α) of a boundary portion between said cylindrical leg portion (2) and said base portion (1).

3. An injection nozzle cap (10) according to claim 1, wherein said cylindrical leg portion (2) is coated on at least an inner surface and/or outer surface thereof with a fluorinated resin film.

## Patentansprüche

1. Einspritzdüsenkappe (10) zur Einpassung in einen Raum zwischen einer Einspritzdüse einer Spritze mit einem zylindrischen, weiblichen Luer-Lockanschluss, und einem weiblichen Luer-Lockanschluss, wobei die Einspritzdüsenkappe (10) einen Basisbereich (1) und einen zylindrischen Bereich (2), der sich abwärts von diesem Basisbereich (1) erstreckt, umfasst, **dadurch gekennzeichnet, dass** auf einer Außenwand des zylindrischen Bereichs (2) im wesentlichen ringförmige, vorgewölbte Bereiche und/oder ein Ring vorgesehen werden.

2. Die Einspritzdüsenkakppe (10) nach Anspruch 1, wobei ein Innendurchmesser (β) eines distalen Endbereichs des zylindrischen Bereichs (2) größer ausgebildet ist, als ein Innendurchmesser (α) eines Grenzbereichs zwischen dem zylindrischen Bereichen (2) und dem Basisbereich (1).

3. Die Einspritzdüsenkappe (10) nach Anspruch 1, wobei der zylindrische Bereich (2) auf mindestens einer inneren Oberfläche und/oder äußeren Oberfläche mit einem fluorierten Harzfilm beschichtet ist.

## Revendications

1. Capuchon de buse d'injection (10) destiné à être inséré de façon ajustée dans un espace entre une buse d'injection d'une seringue munie d'une fixation à verrouillage Luer-Lok femelle cylindrique et une fixation à verrouillage Luer-Lok femelle, ledit capuchon de buse d'injection (10) comprenant une partie de base (1) et une partie de jambe cylindrique (2) s'étendant vers le bas de ladite partie de base (1), **caractérisé en ce que** ladite partie de jambe cylindrique (2) est dotée sur une paroi extérieure de celle-ci d'une partie bombée sensiblement annulaire et/ou d'un anneau.

2. Capuchon de buse d'injection (10) selon la revendication 1, dans lequel un diamètre intérieur (β) d'une partie d'extrémité distale de ladite partie de jambe cylindrique (2) est supérieur à un diamètre intérieur (α) d'une partie de frontière entre ladite partie de jambe cylindrique (2) et ladite partie de base (1).

3. Capuchon de buse d'injection (10) selon la revendication 1, dans lequel ladite partie de jambe cylindrique (2) est revêtue, sur au moins une surface intérieure et/ou surface extérieure de celle-ci, d'un film de résine fluorée.
